# EUROPEAN PATENT SPECIFICATION

(11) **EP 2 537 493 B1**
(45) Date of publication and mention of the grant of the patent: **21.09.2016**
(21) Application number: 11744449.7
(22) Date of filing: 11.01.2011
(51) Int. Cl.: A61F 6/06, A61B 17/00, A61B 17/42, A61F 5/37, A61F 6/08

(54) **PESSARY**
PESSAR
PESSAIRE

(30) Priority: 19.02.2010 JP 2010034202
(43) Date of publication of application: 26.12.2012
(73) Proprietor: Ishii, Kenji, Sayama-shi, Saitama 350-1305 (JP)
(72) Inventor: Ishii, Kenji, Sayama-shi, Saitama 350-1305 (JP)
(74) Representative: Hocking, Adrian Niall
(86) International application number: PCT/JP2011/050262
(87) International publication number: WO 2011/102158

(56) References cited:
- WO-A1-94/13223
- JP-A- 7 503 885
- JP-B- 25 001 420
- JP-Y1- 27 004 688
- US-A1- 2003 149 334

## Description

### FIELD OF THE INVENTION

The present invention relates to a pessary used for treating the prolapse of uterus.

### BACKGROUND OF THE INVENTION

A radical treatment for the prolapse of uterus is an operative treatment. However, the foregoing prolapse of uterus is a disease, which is shown much in older persons, and in addition, it is patient's chronic disease. For this reason, there are some risks to perform a surgical treatment. Moreover, the following cases should be considered; namely, one is a case that a surgical treatment is not made soon, and another is a case that a patient does not hope to accept a surgical treatment. In such cases, there is a need to make a temporal emergency and evacuation treatment until the surgery is carried out.

The foregoing prolapse of uterus is often medically called as the prolapse of genitalia or the prolapse of pelvis organs. This results from the reasons. Usually, it is a rare case that uterus descents resulting from the prolapse of uterus only; in this case, the prolapse of uterus is led in combination with the descensus of bladder or rectum. In general, a name such as the prolapse of uterus has been widely known as a general term of these patient's conditions. Therefore, in this description, a name such as the prolapse of uterus is used as a synonym of the foregoing prolapse of genitalia or pelvis organs.

Conventionally, according to the foregoing case, a pessary is inserted into the vagina of a patient to carry out treatment for easing the symptom of hysteroptosia. A pessary used for the foregoing treatment generally has a shape of ring, and further, various sizes are prepared. The following materials are used as the material quality of the pessary. For example, rigid polyvinyl chloride or polyvinyl chloride having a slight softness (flexibility) by a plasticizer is used (e.g. see website of Smiths Medical Japan Ltd.).

### (URL: http://www.smithsmedical.jp/product/sanfujinka.html)

Examples of existing pessaries are disclosed in US2003/0149334, which is regarded as the closest prior art, and WO94/13223, the cylindrical pessaries being non-deformable in an axial direction but deformable in a radial direction.

### SUMMARY OF THE INVENTION

### Problems to be solved by the Invention

However, if treatment is carried out using the foregoing conventional pessary, the following problem arises. Specifically, if a pessary has a small size, when a patient pushes down, the pessary simply comes out of her vagina. For this reason, of pessary capable of being inserted into patient's vagina, the largest-size pessary or a pessary having a size close thereto must be used.

On the other hand, if a pessary having a certain large size is inserted into the patient's vagina, there is a risk of getting vaginal bleeding, erosion and ulceration by compressing patient's vaginal wall for a long term. If the worst accident is considered, the following cases arise. Specifically, one is a case that a pessary is cut into the vaginal wall; as a result, the pessary is not removed from there. Another is a case that a pore is formed in the vaginal wall; as a result, it communicates with rectum and intracelial wall.

In order to avoid the foregoing cases, the using pessary must be replaced every two or three months to observe patient's vaginal wall. However, when the pessary is attached and detached, a patient has a strong pain, and further, damage is often given to the inlet portion of vagina. Moreover, a soft ring is currently gone on the market as a soft-type pessary. However, this soft ring does not have a sufficiently foldable flexibility. For this reason, when being inserted, the soft ring is deformable in some degree by finger's force, but when being detached, the soft ring is not deformable because a patient can not give force. As a result, there is a risk of giving damage to the inlet portion of vagina. Furthermore, the foregoing soft ring is uniquely formed of a flexible material. For this reason, if an abdominal pressure is applied, the soft ring is deformed; as a result, there is a problem in that the soft ring easily prolapses out of the patient vagina.

The present invention has been made in order to solve the foregoing problems. An object of the invention is to provide a pessary, which can prevent damage to a patient's vaginal wall and reduce patient's dolor when the pessary is inserted and pulled out and used for a long term. Another object of the invention is to provide a pessary, which can easily perform a periodic replacement and long management, and prevent the pessary from readily prolapsing out of patient's vagina by patient's abdominal pressure.

### Means for solving the problems

In order to achieve the foregoing objects, according to the present invention, there is provided a pessary used for treating the prolapse of uterus, characterized by comprising a cylindrical sheet-like member, which is un-deformable in the axial direction but deformable in the radial direction, the sheet-like member including a plurality of first silicone portions and a plurality of second silicone portions. The first silicone portions are discontinuously provided in the circumferential direction at intervals of a gap having a predetermined length. The second silicone portions are provided in a gap between the first silicone portions. The second silicone portions may be softer than the first silicone portions, and deformable in the radial direction.

Furthermore, the sheet-like member of this invention may be formed with a plurality of leg portions, which extend along the axial direction.

### Effects of the invention

According to the present invention, when a pessary is inserted and pulled out, it is possible to prevent damage from being given to patient's vaginal wall, and to reduce patient's dolor. Moreover, in a state that no abdominal pressure is applied while sleeping, a pessary gives no strong compressing to patient's vaginal wall. Therefore, according to the present invention, it is possible to reduce damages given to vaginal wall, which are caused when a rigid pessary is used for a long term.

In addition, according to the present invention, there is no risk such that a pessary simply prolapses out of vagina by patient's abdominal pressure.

As is evident from the foregoing description, this invention has variously excellent effects of easily performing a periodic replacement and long-term management of a pessary.

### BRIEF DESCRIPTION OF DRAWINGS

FIG. 1 is a perspective view showing a pessary not in accordance with the present invention;
FIG. 2 is a perspective view showing a pessary in accordance with the present invention;
FIG. 3 is a view to explain a state that a pessary according to an embodiment of the invention is folded;
FIG. 4 is a view to explain a state that a pessary according to an embodiment of the invention is inserted into a vagina;
FIG. 5 is a perspective view showing another modification example of a pessary of the invention; and
FIG. 6 is a perspective view showing still another modification example of a pessary of the invention.

### BEST MODE FOR CARRYING OUT THE INVENTION

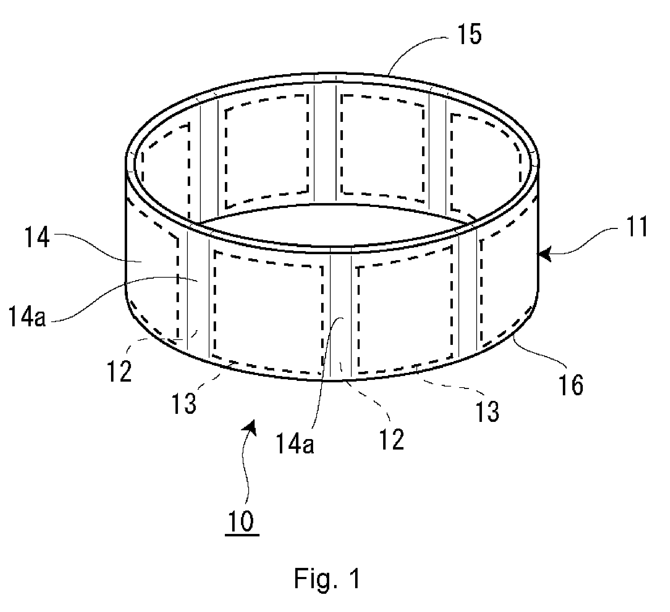

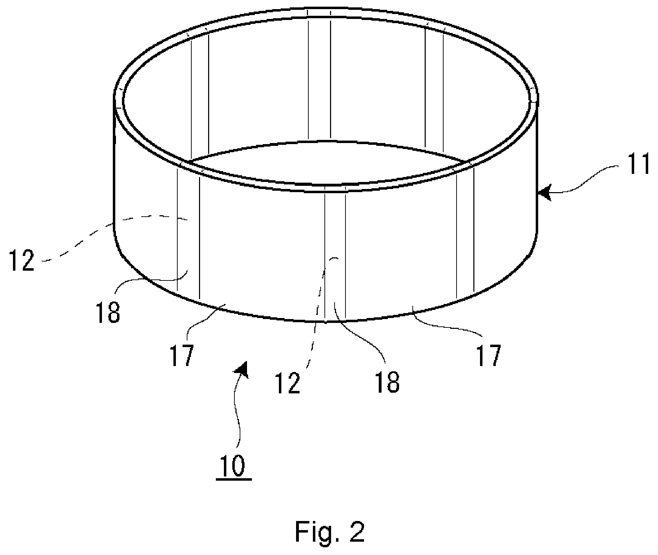

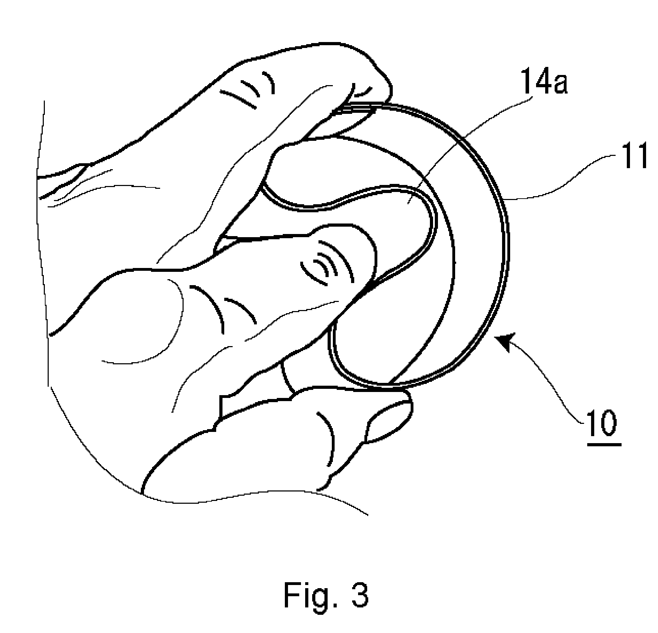

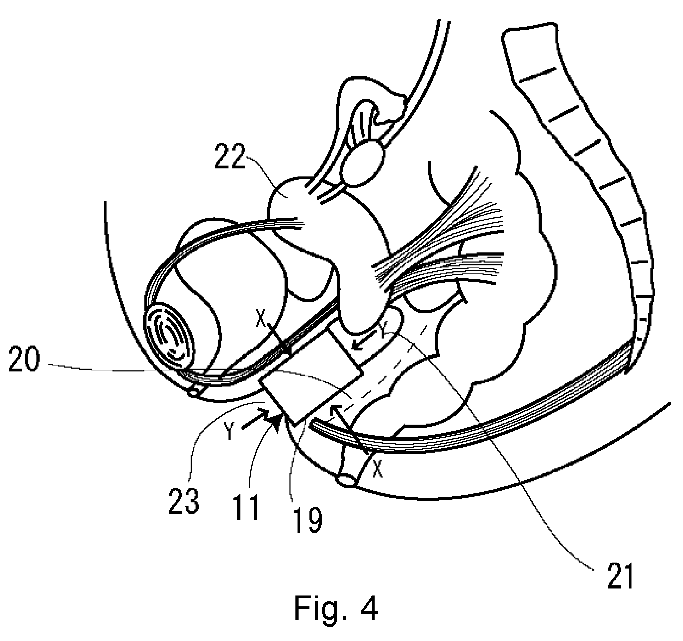

Hereinafter, embodiments of the invention will be described with reference to the accompanying drawings. FIG. 1 is a perspective view showing a pessary according to an embodiment of the invention. FIG. 2 is a perspective view showing a modification example of a pessary of the invention. FIG. 3 is a view to explain a state that a pessary according to an embodiment of the invention is folded. FIG. 4 is a view to explain a state that a pessary according to an embodiment of the invention is inserted into a vagina.

As shown in FIG. 1, a pessary 10 according to this embodiment comprises a flat cylindrical sheet-like member 11, which has a height of about 3 to 4 cm in the axial direction. The foregoing sheet-like member 11 is formed of a plurality of thin metal pieces 13 and silicone portions 14. More specifically, the metal pieces 13 are provided discontinuously in the circumferential direction at intervals of a gap 13 having a predetermined length. The silicone portions 14 are continuously provided in the circumferential direction to coat each metal piece 13. Both end portions 15 and 16 of the sheet-like member 11 re rounded.

In this case, the sheet-like member 11 is not limited to the foregoing structure. For example, as seen from FIG. 2, a sheet-like member 11 may be composed of a plurality of first silicone portions 17 and a plurality of second silicone portions 18. Specifically, the first silicone portions 17 are provided discontinuously in the circumferential direction at intervals of a gap 12. The second silicone portions 18 is softer than the first silicone portions 17 provided in a gap 18 between first silicone portions 17. Moreover, the sheet-like member 11 may be composed of a plurality of metal pieces such that members adjacent to each other are connected like a hinge or wristband of a wrist watch.

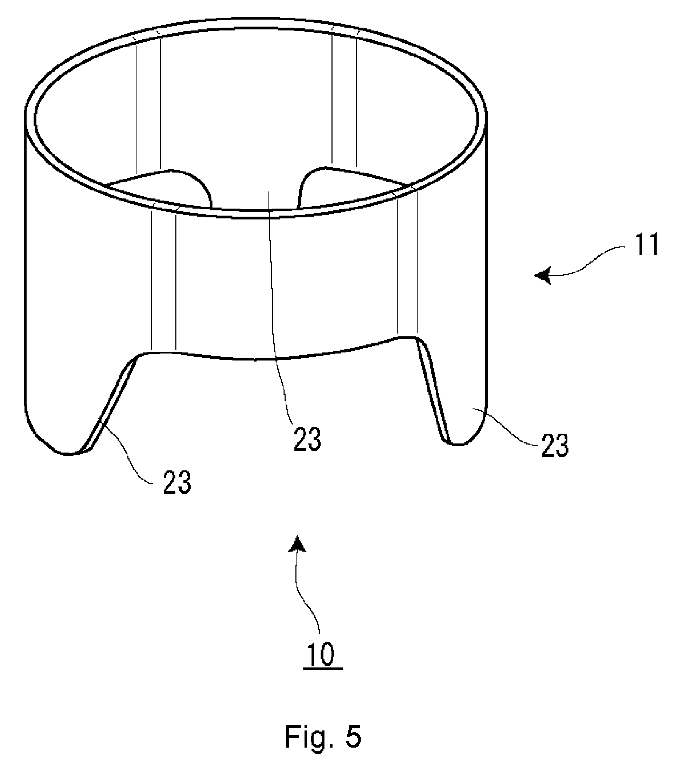

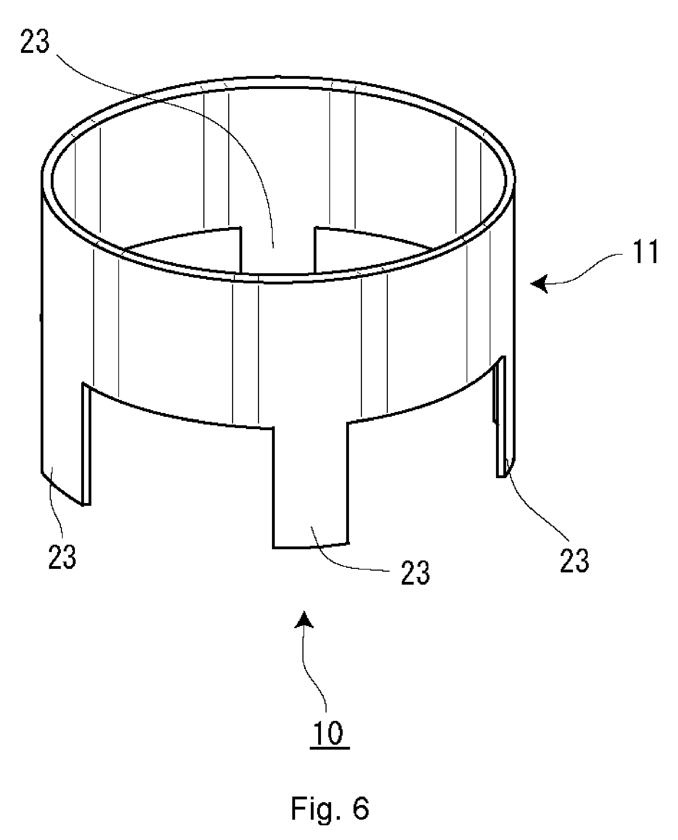

The sheet-like member 11 is not limited to the foregoing material (i.e., silicone). For example, other materials may be used so long as the material has an elasticity, which is un-deformable in the axial direction (i.e. , Y direction in FIG. 4) but deformable in the radial direction (i.e., X direction in FIG. 4). In addition, it is matter of course that the medical safety has been confirmed in the usage of the material. Moreover, the following structures are employed in order to securely prevent a pessary 10 from being rotatably moved in the vagina. For example, according to one structure, the axial height of the sheet-like member 11 is increased as a whole to about 7 to 8 cm, for example. Further, according to another structure, as illustrated in FIG. 5 and FIG. 6 the sheet-like member 11 is formed integrally with a plurality of leg portions 23 (e.g., three in FIG. 5 and four in FIG. 6. As described above, the axial height may be partially increased. This structure is a factor of obtaining the following effect. Namely, the axial height is partially increased, and thereby, this serves to reduce patient's feeling that something happens wrong when a pessary 10 is inserted as compared with the case where the axial height is increased as a whole.

According to this embodiment, the prolapse of uterus is treated using the pessary 10 having the foregoing structure. In this case, as seen from FIG. 3, the silicone portion 14a (or second silicone portion 18) provided in the gap 12 of the sheet-like member 11 is pushed inside in the radial direction with fingers holding a pessary 10. Then, the pessary 10 is set to a state of being folded.

Thereafter, the pessary 10 folded in this manner is inserted into the vagina so that a peripheral wall surface 19 of the sheet-like member 11 contacts with a vaginal wall 20 of a patient. In this case, the pessary 10 can not resist a local bulge of the vaginal wall 20 in the central radial direction (i.e. , X direction in FIG. 4). However, one local bulge of the vaginal wall 20 necessarily presses the sheet-like member 1 against other direction; therefore, it does not stand from the side. This serves to obtain the following advantage.
Specifically, in a vagina 21, the pessary 10 returns from a state of being folded to a state close to the original cylindrical shape by the elasticity of the pessary 10 itself. As a result, the pessary 10 is not kept in a state of being folded when it is inserted. This serves to securely prevent a uterus 22 and a vaginal wall 20 from prolapsing toward a vaginal inlet portion 23. Further, the pessary 10 does not prolapse out of the vagina due to the local bulge of the vaginal wall 20.

Moreover, when being replaced with a new pessary, in the vagina 21, the pessary 10 is set to a state of being folded according to the foregoing procedures. This serves to simply take the pessary 10 out of the vagina.

As is evident from the foregoing description, the pessary 10 according to an embodiment of this invention comprises the sheet-like member having a cylindrical shape, which is un-deformable in the axial direction but deformable in the radial direction. Therefore, it is possible to securely present the prolapse of the uterus 22. Further, the prolapse of uterus can be treated to prevent the pessary 10 from being deformed by the action of an abdominal pressure and from prolapsing out of the vagina. In addition, both sides 15 and 16 of the sheet-like member 11 are rounded. Therefore, this serves to prevent damages to patient's vaginal wall 20 when the pessary 10 is inserted and pulled out, and to reduce patient's dolor.

### Explanation of reference numerals

- 10:: Pessary
- 11:: Sheet-like member
- 12:: Gap
- 13:: Metal pieces
- 14:: Silicone
- 14a:: Silicone portion
- 17:: First silicone
- 18:: Second silicone

## Claims

1. A pessary (10) used for treating the prolapse of uterus , comprising:
a cylindrical sheet-like member (11), which is un-deformable in the axial direction but deformable in the radial direction,
**characterized by** the sheet-like member (11) including: a plurality of first silicone portions (17) discontinuously provided in the circumferential direction at intervals of a gap (12) having a predetermined length; and a plurality of second silicone portions (18) provided in a gap between the first silicone portions (17),
wherein the second silicone portions (18) are softer than the first silicone portions (17), and deformable in the radial direction.

2. The pessary (10) according to claim 1, **characterized in that** the sheet-like member (11) is formed with a plurality of leg portions (23), which extend along the axial direction.

## Patentansprüche

1. Pessar (10), das zur Behandlung eines Prolaps des Uterus verwendet wird und das Folgende umfasst:
ein zylindrisches, bogenartiges Element (11), das in axialer Richtung nicht verformbar, in radialer Richtung aber verformbar ist, **dadurch gekennzeichnet, dass** das bogenartige Element (11) Folgendes umfasst:
eine Vielzahl erster Silikon-Teile (17), die diskontinuierlich in Umfangsrichtung in Intervallen eines Abstands (12), die eine festgelegte Länge aufweist, bereitgestellt werden; und eine Vielzahl zweiter Silikon-Teile (18), die in einem Abstand zwischen den ersten Silikon-Teilen (17) bereitgestellt werden, wobei die zweiten Silikon-Teile (18) weicher als die ersten Silikon-Teile (17) und in radialer Richtung verformbar sind.

2. Pessar (10) nach Anspruch 1, **dadurch gekennzeichnet, dass** das bogenartige Element (11) mit einer Vielzahl von Fußteilen (23) gebildet ist, die sich entlang der axialen Richtung erstrecken.

## Revendications

1. Pessaire (10) utilisé pour traiter le prolapsus de l'utérus, comprenant :
un élément cylindrique en forme de feuille (11) indéformable dans la direction axiale mais déformable dans la direction radiale,
**caractérisé par** l'élément cylindrique en forme de feuille (11) incluant une pluralité de premières portions en silicone (17) présentes de façon discontinue dans la direction circonférentielle à plusieurs intervalles d'un espacement (12) ayant une longueur prédéterminée ; et une pluralité de deuxièmes portions en silicone (18) présentes dans un espacement entre les premières portions en silicone (17), dans lequel les deuxièmes portions en silicone (18) sont plus souples que les premières deuxièmes portions en silicone (17) et sont déformables dans la direction radiale.

2. Pessaire (10) selon la revendication 1 **caractérisé en ce que** l'élément cylindrique en forme de feuille (11) est formé avec une pluralité de portions formant des pattes (23), qui s'étendent le long de la direction axiale.
